Europäisches Patentamt

European Patent Office

Office européen des brevets

(19)

(11) Veröffentlichungsnummer: **0 025 546**
A2

(12)

# EUROPÄISCHE PATENTANMELDUNG

(21) Anmeldenummer: 80105218.4

(22) Anmeldetag: 02.09.80

(51) Int. Cl.³: **A 61 K 45/06,** A 61 K 31/55
// (A61K31/55, 31/54, 31/475,
31/44, 31/40, 31/165, 31/445)

(30) Priorität: 14.09.79 CH 8345/79

(71) Anmelder: **F. HOFFMANN-LA ROCHE & CO.
Aktiengesellschaft, CH-4002 Basel (CH)**

(43) Veröffentlichungstag der Anmeldung: 25.03.81
Patentblatt 81/12

(72) Erfinder: **Lotz, Wolfgang, Dr., Hans-Scherlin-Strasse 11,
D-7801 Ehrenkirchen (DE)**

(84) Benannte Vertragsstaaten: **AT CH DE FR GB IT LI LU NL
SE**

(74) Vertreter: **Lederer, Franz, Dr. et al, Patentanwälte Dr.
Franz Lederer Dipl.-Ing. Reiner F. Meyer-Roxlau
Lucile-Grahn-Strasse 22, D-8000 München 80 (DE)**

(54) Therapeutische Verwendung tranquillisierend wirksamer Benzodiazepinderivate und diese enthaltende
Kombinationspräparate.

(57) Tranquillisierend wirksame Benzodiazepine, insbesondere 1,4-Benzodiazepine, können die Serumprolaktinkonzentration erniedrigen. So kann die durch die Verabreichung von Neuroleptika induzierte Erhöhung der Serumprolaktinkonzentration, welche zu unerwünschten Nebenwirkungen führen kann, dosisabhängig verringert werden, wenn man pro Gewichtsteil Neuroleptikum etwa 0,01 bis 100 Gewichtsteile Benzodiazepinderivat verabreicht, wobei es keine Rolle spielt, ob das Benzodiazepinderivat vor oder nach dem Neuroleptikum oder gleichzeitig mit diesem verabreicht wird. Die gleichzeitige Verabreichung der Wirkstoffe kann als ad hoc Kombination oder in Form einer pharmazeutischen Kombination erfolgen.

2. Sep. 1980

RAN 0025546

F. Hoffmann-La Roche & Co. Aktiengesellschaft,   Basel/Schweiz

Neue therapeutische Verwendung von tranquillisierend wirksamen
Benzodiazepinderivaten, diese enthaltende Kombinationspräparate
sowie deren Herstellung

Die vorliegende Erfindung betrifft eine neue therapeutische
Verwendung von tranquillisierend wirksamen Benzodiazepinderivaten, und zwar die Verwendung von Benzodiazepinen,
welche als minor tranquillizers eingesetzt werden können,
bei der Erniedrigung der Serumprolaktinkonzentration.
Es hat sich nämlich gezeigt, dass die als minor tranquillizers verwendbaren Benzodiazepine nicht nur die zirkulierende
Serumprolaktinkonzentration   erniedrigen können, sondern
insbesondere auch die durch die Verabreichung von Neuroleptika induzierte Erhöhung dieser Hormonkonzentration   dosisabhängig verringern können.

Es ist bekannt, dass erhöhte Serumprolaktinkonzentration
zu unerwünschten Nebenwirkungen führen kann, wie z.B.
Amenorrhoe, Galaktorrhoe  und Gynaecomastie. Wegen ihrer die
Prolaktinfreisetzung inhibierenden Wirkung können solche
Benzodiazepine mit den verschiedensten Neuroleptika kombiniert
werden, wie Tricyclen, insbesondere Phenothiazinderivate,
z.B. Chlorpromazin oder Methopromazin; Rauwolfia-Alkaloide,
z.B. Reserpin; Chinolizidin- oder Indol-Derivate, z.B. Tetrabenazin oder Benzindopyrin; Benzamide, z.B. Sulpirid oder
Metoclopramid; Butyrophenone, z.B. Haloperidol,und dgl.

23.6.80 Kbr/wa

Für die Zwecke der vorliegenden Erfindung geeignete Benzodiazepine sind 1,4-Benzodiazepinderivate,wie 7-Chlor-2-
methylamino-5-phenyl-3H-1,4-benzodiazepin-4-òxyd(Chlordiazepoxyd), 7-Chlor-1,3-dihydro-1-methyl-5-phenyl-2H-1,4-benzo-
diazepin-2-on(Diazepam), 1,3-Dihydro-7-nitro-5-phenyl-2H-
1,4-benzodiazepin-2-on(Nitrazepam), 5-(o-Fluorphenyl)-1,3-
dihydro-1-methyl-7-nitro-2H-1,4-benzodiazepin-2-on(Flunitrazepam), 5-(o-Chlorphenyl)-1,3-dihydro-7-nitro-2H-1,4-benzo-
diazepin-2-on(Clonazepam), 7-Chlor-1,3-dihydro-1-(2-diäthyl-
aminoäthyl)-5-(o-fluorphenyl)-2H-1,4-benzodiazepin-2-on
(Flurazepam), 7-Chlor-2,3-dihydro-1-methyl-5-phenyl-1H-1,4-
benzodiazepin(Medazepam), 7-Brom-1,3-dihydro-5-(2-pyridyl)-
-2H-1,4-benzodiazepin-2-on(Bromazepam), 7-Chlor-5-(o-chlorphenyl)-1,3-dihydro-3-hydroxy-2H-1,4-benzodiazepin-2-on(Lorazepam), 7-Chlor-1,3-dihydro-3-hydroxy-5-phenyl-2H-1,4-benzodiaze-
pin-2-on(Oxazepam), 7-Chlor-1-cyclopropylmethyl-1,3-dihydro-
5-phenyl-2H-1,4-benzodiazepin-2-on(Prazepam), 7-Chlor-1,3-di-
hydro-3-hydroxy-1-methyl-5-phenyl-2H-1,4-benzodiazepin-2-on
(Temazepam), Derivate mit einem weiteren Ring wie z.B. 10-
Chlor-11b-(o-chlorphenyl)-2,3,5,11b-tetrahydrooxazolo[3,2-d]-
[1,4]benzodiazepin-6-(7H)-on(Cloxazolam), 10-Chlor-2,3,5,11b-
tetrahydro-2-methyl-11b-phenyloxazolo[3,2-d][1,4]benzodiazepin-
6-(7H)-on(Oxazolam), 8-Chlor-6-(2-chlorphenyl)-1-methyl-4H-
s-triazolo[4,3-a][1,4]benzodiazepin(Triazolam) sowie deren
Analoga. Sowohl die Neuroleptika als auch die erwähnten Benzodiazepinderivate können gegebenenfalls auch in Form von pharmazeutisch anwendbaren Salzen verwendet werden.


Gegenstand der vorliegenden Erfindung ist demnach die Verwendung eines Benzodiazepinderivates, welches als minor tranquillizer eingesetzt werden kann, bei der Erniedrigung der Serumprolaktinkonzentration, insbesondere bei der Erniedrigung der
durch die Verabreichung von Neuroleptika induzierten Erhöhung
der Serumprolaktinkonzentration.

Wegen der ausgezeichneten Verträglichkeit und geringen Toxizität solcher Benzodiazepinderivate ist nicht mit unerwünschten Nebenwirkungen zu rechnen.

Bei der erfindungsgemässen Verwendung der Benzodiazepinderivate kann das Mengenverhältnis der Wirkstoffe innerhalb relativ weiter Grenzen variieren. Zweckmässigerweise verwendet man pro Gewichtsteil Neuroleptikum etwa 0,01 bis 100 Gewichtsteile, vorzugsweise etwa 0,05 bis 40 Gewichtsteile Benzodiazepinderivat. Ganz besonders bevorzugt werden pro Gewichtsteil Neuroleptikum 1 bis 10 Gewichtsteile Benzodiazepinderivat verwendet.

Die übliche Dosis variiert gemäss dem zu behandelnden Individuum und der jeweiligen Indikation und kann beispielsweise je nach Neuroleptikum 1 bis 100 mg und je nach Benzodiazepinderivat 5 bis 40 mg je Tag bei Menschen bei einer oralen Verabreichung betragen.

Um die erfindungsgemässe Erniedrigung der Serumprolaktinkonzentration zu erreichen, spielt es keine Rolle, ob das Benzodiazepinderivat vor oder nach dem Neuroleptikum, oder gleichzeitig mit diesem verabreicht wird. Wird das Benzodiazepinderivat gleichzeitig mit dem Neuroleptikum verabreicht, so kann dies durch Verabreichung als ad hoc Kombination oder in Form einer pharmazeutischen Kombination erfolgen, welche ein Neuroleptikum bzw. ein pharmazeutisch anwendbares Salz davon und ein Benzodiazepinderivat, welches als minor tranquillizer verwendet werden kann, bzw. ein pharmazeutisch anwendbares Salz davon enthält.

0025546

Das Gewichtsverhältnis von Neuroleptikum zu Benzodiazepinderivat ist in der pharmazeutischen Kombination dasselbe wie
bei ad hoc Kombination der beiden Wirkstoffe.

Pharmazeutische Kombinationen enthaltend ein Neuroleptikum
und ein als minor tranquillizer verwendbares Benzodiazepinderivat sind zum Teil neu und zum Teil bekannt (beispielsweise die Kombination Diazepam/Sulpirid bzw. Bromazepam/Sulpirid). Die neuen Kombinationen sind ebenfalls Gegenstand
der vorliegenden Erfindung.

Ein weiterer Gegenstand der Erfindung betrifft somit eine
noch neue pharmazeutische Kombination von einem Neuroleptikum mit einem Benzodiazepinderivat, welches als minor tranquillizer verwendet werden kann, sowie die Herstellung einer
solchen pharmazeutischen Kombination.

Als Beispiel für eine solche pharmazeutische Kombination
kann die folgende Zusammensetzung angegeben werden.

Man stellt eine Tablette der folgenden Zusammensetzung her:

| | |
|---|---|
| Nitrazepam | 20,0 mg |
| Sulpirid | 12,5 mg |
| Lactose | |
| Stärke | ad 350 mg. |
| Talk und | |
| Magnesiumstearat | |

Die vorteilhaften Eigenschaften der erfindungsgemässen pharmazeutischen Kombinationen sowie die die Prolaktinfreisetzung
inhibierende Wirkung der als minor tranquillizer verwendbaren Benzodiazepine sollen anhand der nachfolgend wiedergegebenen
Versuche gezeigt werden.


Ratten männlichen Geschlechts werden während 4 Tagen an die
orale Behandlung mit einer Schlundsonde durch Applikation
von je 0,2 ml physiologischer Kochsalzlösung gewöhnt. Die
Testsubstanzen werden in 0,2 ml eines "Standard Suspending
Vehicle"(SSV) bestehend aus 0,5 % Carboxymethylcellulose,
0,4 % Tween 80 (Polyoxyethylene-sorbitan-fettsäureester),
0,9 % Benzylalkohol und 0,9 % Natriumchlorid in Wasser suspendiert und mit der gleichen Schlundsonde gemäss dem Zeitschema,das in den Tabellen 1 und 2 angegeben ist, appliziert. Zur angegebenen Zeit werden die Tiere unter Vermeidung von Stress, der zu unspezifischen Erhöhungen der Serumprolaktinkonzentrationen führt, dekapitiert. Blut wird aus
dem Rumpf gesammelt, und das daraus gewonnene Serum bis zur
Hormonbestimmung tiefgefroren. Die Prolaktinkonzentrationen
werden mit Hilfe eines spezifischen Radioimmunoassays bestimmt. Die Ergebnisse sind in (ng/ml) ausgedrückt. Die
statistischen Analysen werden mit einem verteilungsunabhängigen Test (Kolmogorov-Smirnov Test) und in mehreren Fällen
zusätzlich oder nur mit dem Studence t-Test durchgeführt. Die
in den folgenden Tabellen angegebenen Werte stellen Mittelwerte dar mit gleichzeitiger Angabe des Fehlers vom Mittelwert (Standard Error of the Mean; SEM).

Tabelle 1

| Wirkstoffe p.o. (mg/kg) | | n | Serumprolaktin-konzentr. (ng/mL) | P=* | P<** |
|---|---|---|---|---|---|
| 0 min | 15 min | | 30 min | | |
| SSV | SSV | 13 | $17 \pm 4$ | | |
| Sulpirid (1) | SSV | 11 | $92 \pm 11$ | | |
| Sulpirid (1) | Nitrazepam (10) | 12 | $47 \pm 6$ | 0.0002 | 0.001 |
| 0 min | 15 min | | 60 min | | |
| SSV | SSV | 12 | $13 \pm 2$ | | |
| Sulpirid (1) | SSV | 12 | $50 \pm 7$ | | |
| Sulpirid (1) | Nitrazepam (10) | 12 | $22 \pm 2$ | 0.008 | 0.001 |
| 0 min | 30 min | | 45 min | | |
| SSV | SSV | 14 | $17 \pm 3$ | | |
| Nitrazepam (10) | SSV | 14 | $5 \pm 0.2$ | 0.0000 | 0.001 |
| SSV | Sulpirid (1) | 14 | $121 \pm 12$ | | |
| Nitrazepam (10) | Sulpirid (1) | 14 | $9 \pm 2$ | 0.0000 | 0.001 |
| Flunitra-zepam (10) | Sulpirid (1) | 7 | $15 \pm 5$ | 0.0000 | 0.001 |
| Chlordia-zepoxid (10) | Sulpirid (1) | 7 | $45 \pm 9$ | 0.0034 | 0.001 |
| SSV | Haloperidol (1) | 7 | $73 \pm 14$ | | |
| Nitrazepam (10) | Haloperidol (1) | 7 | $4 \pm 0.3$ | 0.0006 | 0.001 |
| SSV | SSV | 7 | $18 \pm 5$ | | |
| Medazepam (100) | SSV | 7 | $5 \pm 0.3$ | 0.0082 | 0.05 |
| SSV | Sulpirid (1) | 7 | $127 \pm 8$ | | |
| Medazepam (1) | Sulpirid (1) | 7 | $138 \pm 15$ | n.s. | n.s. |
| Medazepam (10) | Sulpirid (1) | 7 | $29 \pm 8$ | 0.0012 | 0.001 |
| Medazepam (100) | Sulpirid (1) | 7 | $17 \pm 4$ | 0.0012 | 0.001 |

Tabelle 2

| Wirkstoffe p.o. (mg/kg) 0 Min | n | Serumprolaktin- konzentr. (ng/ml) 30 Min | P=* | P<** |
|---|---|---|---|---|
| SSV | 7 | 6 ± 1 | | |
| Haloperidol (1) | 7 | 73 ± 14 | | |
| Haloperidol(1) + Nitrazepam(10) | 7 | 23 ± 5 | n.s. | 0.01 |
| Chlorpromazin (20) | 7 | 54 ± 10 | | |
| Chlorpromazin (20)+Nitrazepam(10) | 7 | 32 ± 7 | n.s. | 0.05 |
| SSV | 7 | 15 ± 2 | | |
| Nitrazepam (0.1) | 7 | 16 ± 5 | n.s. | − |
| Nitrazepam (1) | 7 | 10 ± 1 | n.s. | − |
| Sulpirid (1) | 10 | 146 ± 11 | | |
| Sulpirid (1) + Nitrazepam (0.1) | 7 | 172 ± 12 | n.s. | − |
| Sulpirid (1) + Nitrazepam (1) | 7 | 75 ± 7 | 0.0150 | − |
| Sulpirid (1) + Nitrazepam (10) | 7 | 48 ± 11 | 0.0001 | − |
| SSV | 13 | 19 ± 3 | | |
| Sulpirid (1) | 18 | 203 ± 20 | | |
| Sulpirid (1)+ Diazepam (0.01) | 7 | 231 ± 20 | n.s. | − |
| Sulpirid (1)+ Diazepam (0.1) | 7 | 211 ± 21 | n.s. | − |
| Sulpirid (1)+ Diazepam (1) | 14 | 224 ± 17 | n.s. | − |
| Sulpirid (1)+ Diazepam (10) | 12 | 87 ± 11 | 0.0002 | − |
| Sulpirid (1)+ Chlord. (0.01) | 7 | 187 ± 24 | n.s. | − |
| Sulpirid (1)+ Chlord. (0.1) | 7 | 202 ± 14 | n.s. | − |
| Sulpirid (1)+ Chlord. (1) | 14 | 132 ± 12 | 0.022 | − |
| Sulpirid (1)+ Chlord. (10) | 11 | 97 ± 17 | 0.097 | − |

Fortsetzung von Tabelle 2

| | | | | |
|---|---|---|---|---|
| SSV | 7 | 13 ± 2 | | – |
| Haloperidol (1) | 7 | 312 ± 80 | | – |
| Haloperidol (1) + Diazepam (10) | 7 | 17 ± 2 | | 0.01 |
| Thioridazin (10) | 7 | 131 ± 52 | | – |
| Thioridazin (10) + Diazepam (10) | 7 | 17 ± 1 | | 0.05 |
| Metoclopramid (1) | 7 | 297 ± 151 | | – |
| Metoclopramid (1) + Diazepam (10) | 7 | 54 ± 12 | | 0.05 |
| Reserpin (1) | 7 | 69 ± 11 | | – |
| Reserpin (1) + Diazepam (10) | 7 | 20 ± 5 | | 0.01 |
| SSV | 7 | 15 ± 2 | | – |
| Sulpirid (1) | 7 | 499 ± 81 | | – |
| Sulpirid (1) + Temesta (10) | 7 | 298 ± 19 | | 0.05 |
| Sulpirid (1) + Triazolam (10) | 7 | 135 ± 39 | | 0.01 |
| Sulpirid (1) + Bromazepam (10) | 7 | 62 ± 16 | | 0.001 |

0025546

Ratten weiblichen Geschlechts werden während 10 Tagen
1 bzw. 10 mg/kg der Testsubstanz in 0,2 ml SSV oral verabreicht. 30 Minuten nach der letzten Applikation werden
die Tiere unter Vermeidung von Stress dekapitiert, und
die Serumprolaktinkonzentrationen, wie oben beschrieben,
bestimmt.

Tabelle 3

| Benzodiazepinderivat (mg/kg) | n | Prolaktin (ng/ml) | P=* |
|---|---|---|---|
| SSV | 9 | 32 ± 8 | − |
| Chlordiazepoxid (1) | 5 | 30 + 13 | n.s. |
| Chlordiazepoxid (10) | 5 | 15 ± 4 | n.s. |
| Diazepam (1) | 5 | 21 ± 5. | n.s. |
| Diazepam (10) | 5 | 7 ± 0.9 | 0.003 |
| Nitrazepam (1) | 5 | 11 ± 2 | n.s. |
| Nitrazepam (10) | 5 | 4 ± 0.3 | 0.003 |
| Bromazepam (1) | 5 | .13 ± 3 | n.s. |
| Bromazepam (10) | 5 | 6 ± 1 | 0.001 |
| Clonazepam (1) | 5 | 7 ± 1 | 0.001 |
| Flunitrazepam (1) | 5 | 9 ± 1 | 0.01 |
| Flunitrazepam (10) | 5 | 7 ± 0.8 | 0.001 |

\* Kolmogorov-Smirnov Test
\*\* t-Test

n.s. = statistisch nicht signifikant
SSV = Standard Suspending Vehicle

0025546

Die obigen, im Tierversuch ermittelten Ergebnisse konnten
im nachfolgend beschriebenen klinischen Versuch bestätigt
werden.

6 männliche Volontäre, die über alle Vorgänge und möglichen
Komplikationen des Experimentes informiert worden waren und
ihr Einverständnis dazu gegeben hatten, wurden vor dem Experiment einer gründlichen Untersuchung inklusive Bestimmung der hämatologischen Werte sowie biochemischen Analyse von Blut und Urin unterworfen. 30 Minuten vor dem Experiment wurde jeweils eine Flügelkanüle in die Armvene des
Probanden gelegt. 30 Minuten später wurde Blut zur Bestimmung
der basalen Prolaktinkonzentration entnommen. Zur Zeit 0
wurde Sulpirid i.m. und Placebo oral verabreicht. Nach 15,
30, 60, 90, 120, 180 und 240 Minuten wurde jeweils eine venöse
Blutprobe entnommen. Am nächsten Tag wurde mit denselben
Volontären das gleiche Experiment wiederholt, jedoch wurde
die orale Placebogabe durch 20 mg Nitrazepam ersetzt. Am Beispiel der Kombination von 6,25 mg Sulpirid i.m. und 20 mg
Nitrazepam p.o. sollen die Ergebnisse erläutert werden.

Tabelle 4

| Zeit | Serumprolaktinkonzentration | |
|------|-----------------------------|--------------------------------------------|
|      | 6.25 mg Sulpirid i.m.       | 6.25 mg Sulpirid i.m. + 20 mg Nitrazepam p.o. |
| -.30' | 10.0 ± 1.2 | 16.1 ± 2.4 |
| 15'  | 52.2 ± 4.2 | 30.0 ± 4.5 |
| 30'  | 50.5 ± 1.9 | 36.0 ± 5.2 |
| 60'  | 46.4 ± 4.0 | 29.7 ± 4.8 |
| 90'  | 39.7 ± 1.9 | 26.5 ± 3.2 |
| 120' | 36.4 ± 4.8 | 23.8 ± 3.9 |
| 180' | 28.2 ± 4.2 | 24.8 ± 3.6 |
| 240' | 23.0 ± 3.8 | 20.3 ± 2.8 |

Wie aus der obigen Tabelle 4 hervorgeht, erhöhen 6,25 mg
Sulpirid i.m. die Serumprolaktinkonzentration von 10 ± 1,2
im Mittel bei -30 Min. auf 52,2 ± 4,2 ng/ml 15 Minuten nach
Verabreichung des Sulpirid. Die gleichzeitige orale Verabreichung von 20 mg Nitrazepam p.o. erniedrigt die Prolaktinfreisetzung in einem solchen Masse, dass die mittlere
Konzentration von 16,1 ± 2,4 bei -30 Min. auf nur 30 ± 4,5
ng/ml 15 Minuten nach der Sulpiridverabreichung ansteigt.

Erfindungsgemäss wird im allgemeinen sowohl das Neuroleptikum wie auch das Benzodiazepinderivat oral verabreicht.
Das gleiche gilt natürlich auch für die erfindungsgemässen
Kombinationen, welche z.B. in Form von Tabletten, Lacktabletten, Dragées, Hart- und Weichgelatinekapseln, Lösungen,
Emulsionen oder Suspensionen, verabreicht werden können.
Die Verabreichung kann aber auch rektal, z.B. in Form von
Suppositorien, lokal oder perkutan, z.B. in Form von Salben,
Crèmes, Gelées, Lösungen, oder parenteral, z.B. in Form von
Injektionslösungen, erfolgen.

Zur Herstellung von Tabletten, Lacktabletten, Dragées und
Hartgelatinekapseln können die Wirkstoffe mit pharmazeutisch
inerten, anorganischen oder organischen Excipientien
verarbeitet werden. Als solche Excipientien kann man für
Tabletten, Dragées und Hartgelatinekapseln z.B. Lactose,
Maisstärke oder Derivate davon, Talk, Stearinsäure oder deren
Salze etc. verwenden.

Für Weichgelatinekapseln eignen sich als Excipientien z.B.
vegetabile Oele, Wachse, Fette, halbfeste oder flüssige Polyole etc.; je nach Beschaffenheit des Wirkstoffes sind jedoch
bei Weichgelatinekapseln überhaupt keine Excipientien erforderlich.

Zur Herstellung von Lösungen und Sirupen eignen sich als Excipientien z.B. Wasser, Polyole, Saccharose, Invertzucker,
Glukose und dergleichen.

Für Injektionslösungen eignen sich als Excipientien z.B.
Wasser, Alkohole, Polyole, Glyzerin, vegetabile Oele etc.

Für Suppositorien, lokale oder perkutane Anwendungsformen
eignen sich als Excipientien z.B. natürliche oder gehärtete
Oele, Wachse, Fette, halbflüssige oder flüssige Polyole
und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.
Sie können auch noch andere therapeutisch wertvolle Stoffe
enthalten.

Patentansprüche

1.    Verwendung von tranquillisierend wirksamen Benzodiazepinderivaten bei der Erniedrigung der Serumprolaktinkonzentration.

2.    Verwendung eines Benzodiazepinderivates, welches als minor tranquillizer eingesetzt werden kann, bei der Erniedrigung der durch die Verabreichung von Neuroleptika induzierten Erhöhung der Serumprolaktinkonzentration.

3.    Verwendung nach Anspruch 1 oder 2, dadurch gekennzeichnet, dass das Benzodiazepinderivat ein 1,4-Benzodiazepinderivat ist.

4.    Verwendung nach Anspruch 3, dadurch gekennzeichnet, dass das 1,4-Benzodiazepinderivat Chlordiazepoxyd, Diazepam, Flunitrazepam, Clonazepam, Flurazepam, Medazepam, Bromazepam, Lorazepam, Oxazepam, Prazepam, Temazepam, Cloxazolam, Oxazolam oder Triazolam ist.

5.    Verwendung nach Anspruch 3, dadurch gekennzeichnet, dass das 1,4-Benzodiazepinderivat Nitrazepam ist.

6.    Verwendung nach einem der Ansprüche 2 bis 5, dadurch gekennzeichnet, dass das Neuroleptikum ein Tricyclus, insbesondere ein Phenothiazin-Derivat, wie Chlorpromazin oder Methopromazin; ein Rauwolfia-Alkaloid, wie Reserpin; ein Chinolizidin- oder Indol-Derivat, wie Tetrabenazin oder Benzindopyrin; ein Benzamid, wie Sulpirid oder Metoclopramid; oder ein Butyrophenon, wie Haloperidol, ist.

7.    Verwendung nach Anspruch 6, dadurch gekennzeichnet, dass das Neuroleptikum Sulpirid oder Haloperidol
ist.

8.    Verwendung nach einem der Ansprüche 2 bis 7,
dadurch gekennzeichnet, dass man pro Gewichtsteil Neuroleptikum etwa 0,01 bis 100 Gewichtsteile, vorzugsweise etwa
0,05 bis 40 Gewichtsteile, Benzodiazepinderivat verwendet.

9.    Verwendung nach Anspruch 8, dadurch gekennzeichnet, dass man pro Gewichtsteil Neuroleptikum 1 bis 10 Gewichtsteile Benzodiazepinderivat verwendet.

10.    Verwendung nach einem der Ansprüche 2 bis 9,
dadurch gekennzeichnet, dass das Benzodiazepinderivat vor
dem Neuroleptikum verabreicht wird.

11.    Verwendung nach einem der Ansprüche 2 bis 9,
dadurch gekennzeichnet, dass das Benzodiazepinderivat nach
dem Neuroleptikum oder gleichzeitig mit diesem verabreicht
wird.

12.    Verwendung nach Anspruch 11, dadurch gekennzeichnet, dass das Benzodiazepinderivat zusammen mit dem Neuroleptikum in Form einer pharmazeutischen Kombination verabreicht wird.

13. Pharmazeutische Kombination enthaltend ein tranquillisierend wirksames Benzodiazepinderivat sowie ein Neuroleptikum mit Ausnahme der Kombinationen Diazepam/Sulpirid und Bromazepam/Sulpirid.

14. Kombination nach Anspruch 13, dadurch gekennzeichnet, dass das Benzodiazepinderivat ein 1,4-Benzodiazepinderivat ist.

15. Kombination nach Anspruch 14, dadurch gekennzeichnet, dass das 1,4-Benzodiazepinderivat Chlordiazepoxyd, Diazepam, Flunitrazepam, Clonazepam, Flurazepam, Medazepam, Bromazepam, Lorazepam, Oxazepam, Prazepam, Temazepam, Cloxazolam, Oxazolam oder Triazolam ist.

16. Kombination nach Anspruch 14, dadurch gekennzeichnet, dass das 1,4-Benzodiazepinderivat Nitrazepam ist.

17. Kombination nach einem der Ansprüche 13 bis 16, dadurch gekennzeichnet, dass das Neuroleptikum ein Tricyclus, insbesondere ein Phenothiazin-Derivat, wie Chlorpromazin oder Methopromazin; ein Rauwolfia-Alkaloid, wie Reserpin; ein Chinolizidin- oder Indol-Derivat, wie Tetrabenazin oder Benzindopyrin; ein Benzamid, wie Sulpirid oder Metoclopramid; oder ein Butyrophenon, wie Haloperidol, ist.

18. Kombination nach Anspruch 17, dadurch gekennzeichnet, dass das Neuroleptikum Sulpirid oder Haloperidol ist.

19. Kombination nach einem der Ansprüche 13 bis 18, dadurch gekennzeichnet, dass sie pro Gewichtsteil Neuroleptikum etwa 0,01 bis 100 Gewichtsteile, vorzugsweise etwa 0,05 bis 40 Gewichtsteile, Benzodiazepinderivat enthält.

0025546

20.    Kombination nach Anspruch 19, dadurch gekennzeichnet, dass sie pro Gewichtsteil Neuroleptikum
1 bis 10 Gewichtsteile Benzodiazepinderivat enthält.


***

<u>Patentansprüche</u>   für Oesterreich

1.   Verfahren zur Herstellung einer pharmazeutischen Kombination enthaltend ein tranquillisierend
wirksames Benzodiazepinderivat sowie ein Neuroleptikum
mit Ausnahme der Kombinationen Diazepam/Sulpirid und
Bromazepam/Sulpirid, dadurch gekennzeichnet, dass man die
Wirkstoffe in eine galenische Darreichungsform bringt.

2.   Verfahren nach Anspruch 1, dadurch gekennzeichnet, dass man als Benzodiazepinderivat ein 1,4-
Benzodiazepinderivat verwendet.

3.   Verfahren nach Anspruch 2, dadurch gekennzeichnet, dass man als 1,4-Benzodiazepinderivat Chlordiazepoxyd, Diazepam, Flunitrazepam, Clonazepam, Flurazepam, Medazepam, Bromazepam, Lorazepam, Oxazepam, Prazepam,
Temazepam, Cloxazolam, Oxazolam oder Triazolam verwendet.

4.   Verfahren nach Anspruch 2, dadurch gekennzeichnet, dass man als 1,4-Benzodiazepinderivat Nitrazepam verwendet.

5.   Verfahren nach einem der Ansprüche 2 bis 4,
dadurch gekennzeichnet, dass man als Neuroleptikum ein Tricyclus, insbesondere ein Phenothiazin-Derivat, wie Chlorpromazin oder Methopromazin; ein Rauwolfia-Alkaloid, wie
Reserpin; ein Chinolizidin- oder Indol-Derivat, wie Tetrabenazin oder Benzindopyrin; ein Benzamid, wie Sulpirid
oder Metoclopramid; oder ein Butyrophenon, wie Haloperidol,
verwendet.

6.   Verfahren nach Anspruch 5, dadurch gekennzeichnet, dass man als Neuroleptikum Sulpirid oder
Haloperidol verwendet.

00255 46

7. Verfahren nach einem der Ansprüche 2 bis 6, dadurch gekennzeichnet, dass man pro Gewichtsteil Neuroleptikum etwa 0,01 bis 100 Gewichtsteile, vorzugsweise etwa 0,05 bis 40 Gewichtsteile, Benzodiazepinderivat verwendet.

8. Verfahren nach Anspruch 7, dadurch gekennzeichnet, dass man pro Gewichtsteil Neuroleptikum 1 bis 10 Gewichtsteile Benzodiazepinderivat verwendet.

\*\*\*